# EUROPEAN PATENT SPECIFICATION

(11) **EP 3 703 802 B1**
(45) Date of publication and mention of the grant of the patent: **16.10.2024**
(21) Application number: 18808487.5
(22) Date of filing: 30.10.2018
(51) Int. Cl.: A61M 11/02, A61M 15/08, A61M 19/00, A61B 17/24, A61M 5/46, A61M 25/00

(54) **NASAL DELIVERY DEVICES AND METHODS OF USE THEREOF**
VORRICHTUNGEN ZUR NASALEN VERABREICHUNG UND VERWENDUNGSVERFAHREN DAFÜR
DISPOSITIFS D'ADMINISTRATION NASALE ET LEURS PROCÉDÉS D'UTILISATION

(30) Priority: 31.10.2017 US 201762579723 P
(43) Date of publication of application: 09.09.2020
(73) Proprietor: Clexio Biosciences Ltd., Jerusalem 9123901 (IL)
(72) Inventor: CHAN, Philip, Skillman, New Jersey 08558 (US); ERDOSI, Ernest, Milltown, New Jersey 08850 (US); KIRKPATRICK, JR, Alan D., Newtown, Pennsylvania 18940 (US); MAJEWSKI, John P., San Mateo, California 94402 (US); WEITZEL, Douglas, Duluth, Georgia 30097 (US); CARLSON, Morgan, Nashua, New Hampshire 03064 (US); LONGAN, John, Nashua, New Hampshire 03063 (US); RUDOLPH, James, Saint Joseph, Michigan 49085-2336 (US)
(74) Representative: D Young & Co LLP
(86) International application number: PCT/IB2018/058500
(87) International publication number: WO 2019/087071

(56) References cited:
- KR-A- 20170 114 150
- US-A1- 2013 158 475
- US-A1- 2015 290 439
- US-A1- 2016 361 507
- US-A1- 2017 296 759

## Description

### TECHNICAL FIELD

The present disclosure relates to nasal delivery devices, and more particularly, to improved nasal delivery devices and methods for self-administering medicament.

### BACKGROUND

Certain types of pain associated with headaches and facial aches are known to respond to treatment by the direct application of anesthetic to the sphenopalatine ganglion or its surrounding region. The sphenopalatine ganglion (SPG) is a nerve bundle located toward the center of the head. The SPG is bilateral, and one is located behind each side of the nose. The SPG plays a unique role in headache disorders as a key structure responsible for the expression of cranial autonomic symptoms, most commonly seen in trigeminal autonomic cephalalgia. Cluster headache, which is one type of trigeminal autonomic cephalalgia, is a severe headache characterized by recurrent episodes of excruciating pain, often on one side of the head.

Conventional methods for treating pain associated with headaches and facial aches have many safety and efficacy problems, and patients experiencing severe pain are typically unable to travel to a physician's office for treatment before the pain intensifies. It is especially difficult to mitigate a full-blown cluster headache attack if the pain is not treated when the headache begins.

Although the anatomical position of the SPG makes it difficult to treat with a local anesthetic, a nerve block of the SPG has been known to provide effective relief in a variety of pain conditions. With this approach, anesthetic is applied to the SPG or its surrounding area by a trained medical professional, who typically inserts a cotton-tipped applicator soaked in the anesthetic into the nostril of the patient. Nasal catheter devices have also been used by physicians to administer medicament to the SPG or its surrounding area. The success of these methods depends heavily on the skill and experience of the physician, and patients experiencing severe pain are typically unable to receive treatment from a physician before the pain escalates.

Therefore, there is a need for improved nasal delivery devices and methods that enable patients to apply medicament on-demand for fast relief from head or facial pain, and to lessen or prevent reoccurrence. Document US2013/158475 A1 discloses an example of nasal delivery devices and methods that enable patients to apply medicament on-demand.

### SUMMARY

Painful episodes associated with facial aches and headaches, such as cluster headaches and migraines, occur at varying frequencies from patient to patient. When a cluster headache occurs, it often takes too long for a patient to travel to a physician in order to receive local administration of medicament to the SPG, or its surrounding area, in order to prevent or mitigate a full-blown cluster headache attack. Therefore, it is desirable for a patient to be able to self-administer the medicament locally to the SPG or surrounding area using a compact and portable device for treating the cluster headache in a home setting, without the need of a physician or other trained medical professional.

A challenge for self-administered intranasal delivery devices is to ensure a safe and substantially pain-free insertion without having a trained professional position the delivery device and administer the medicament. There are several aspects of the present invention that help ensure proper positioning and medicament delivery, including, for example: a device capable of (1) sensing, via integrated sensors, a clear sinus passageway and autonomously steering itself into position; and/or (2) providing an external datum and direction-finding system to guide the patient in positioning the device so that insertion may be effected without the additional need for steering the catheter; and/or (3) steering itself automatically, and storing all motions during a training session performed by, and controlled by, a trained medical professional such that the device can subsequently "replay" the motions automatically when needed during self-administration.

In an aspect of the present invention, there is provided an intranasal device for the self-administration of medicament includes a body, a catheter, a feedback mechanism, and a delivery system. The body has a chamber to store the medicament. The catheter is configured to extend from the body and is in fluid communication with the chamber. A distal end of the catheter is configured for entry into a nasal passage of a patient. The feedback mechanism is configured to indicate a position of the distal end of the catheter within the nasal passage. The delivery system is configured to deliver the medicament from the chamber into the nasal passage via the catheter, wherein the delivery system comprises a centrifugal pump, a peristaltic pump, a piezoelectric pump, a motorized syringe, a pneumatically controlled syringe, a hydraulically controlled syringe, a pressurized reservoir using a software controlled valve arrangement or combinations thereof.

As used herein, delivery or administration of medicament "toward the SPG" and similar such phrases are intended to include the SPG itself and/or the surrounding region in proximity to the SPG, e.g., the pterygopalatine fossa which houses the SPG, which may include nearby tissue or mucous membranes.

All manner of medicaments suitable for introduction at or in the vicinity of the SPG are contemplated for use in accordance with the present invention. The term medicament is used herein to refer to a pharmaceutical formulation comprising one or more pharmaceutically active compound(s). Examples of suitable medicaments in accordance with the present invention include those that alleviate or eliminate pain associated with a facial ache or headache (e.g., cluster headache or migraine), such as anesthetics. Suitable medicaments may comprise one or more pharmaceutically active compound(s) selected from the group comprising or consisting of lidocaine, zolmitriptan, sumatriptan, ambucaine, amolanone, amylocaine, benoxinate, betoxycaine, biphenamine, bupivacaine, butacaine, butamben, butanilicicaine, butethamine, butoxycaine, carticaine, cocaethylene, cocaine, cyclomethycaine, dibucaine, dimethisoquin, dimethocaine, diperodon, dyclonine, ecgonidine, ecgonine, ethyl aminobenzoate, ethyl chloride, etidocaine, eucaine, euprocin, fenalcomine, fomocaine, hexylcaine, hydroxyprocaine, hydroxytetracaine, isobutyl p-aminobenzoate, leucinocaine mesylate, levoxadrol, meperidine, mepivacaine, meprylcaine, metabutoxycaine, methyl chloride, myrtecaine, naepaine, octacaine, orthocaine, oxethazaine, parethoxycaine, phenacaine, phenol, a pipecoloxylidide, piperocaine, piridocaine, polidocanol, pramoxine, sameridine, prilocaine, propanocaine, proparacaine, propipocaine, propoxycaine, pseudococaine, pyrrocaine, quinine urea, risocaine, ropivacaine, salicyl alcohol, tetracaine, tolycaine, trimecaine, veratridine, zolamine, all pharmaceutically acceptable salts thereof, and combinations thereof.

According to an exemplary embodiment, the medicament comprises lidocaine or a pharmaceutically acceptable salt thereof, wherein the medicament is used to treat, for example, pain associated with cluster headache.

Non-limiting examples of pain that may be treated by methods of the present invention include: cluster headache, sphenopalatine neuralgia, migraine headache, atypical facial pain, cancer pain of the head and/or neck, tongue and/or mouth pain, temporomandibular joint (TMJ) pain, Sluder's neuralgia, paroxysmal hemicranias, herpes zoster, postherpetic neuralgia, vasomotor rhinitis, complex regional pain syndrome (CRPS), reflex sympathetic dystrophy (RSD), lower back pain, post-traumatic headache, fibromyalgia and postdural puncture headache.

As used herein, the term therapeutically effective amount may refer to an amount that, when administered to a particular subject, achieves a therapeutic effect by inhibiting, alleviating or curing a disease, disorder or symptom(s) in the subject or by prophylactically inhibiting, preventing or delaying the onset of a disease, disorder or symptom(s). A therapeutically effective amount may be an amount which relieves to some extent one or more symptoms of a disease or disorder in a subject; and/or returns to normal either partially or completely one or more physiological or biochemical parameters associated with or causative of the disease or disorder; and/or reduces the likelihood of the onset of the disease, disorder or symptom(s). According to one embodiment, a therapeutically effective amount is an amount which achieves a therapeutic effect by alleviating or eliminating pain associated with a facial ache or headache, such as a cluster headache or migraine headache, e.g., by administering a medicament comprising lidocaine or a pharmaceutically acceptable salt thereof.

This summary is provided to introduce a selection of concepts in a simplified form that are further described below in the Detailed Description. This Summary is not intended to identify key features or essential features of the claimed subject matter, nor is it intended to be used to limit the scope of the claimed subject matter. Furthermore, the claimed subject matter is not constrained to limitations that solve any or all disadvantages noted in any part of this disclosure.

### BRIEF DESCRIPTION OF THE DRAWINGS

A more detailed understanding may be had from the following description, given by way of example in conjunction with the accompanying drawings, wherein:
FIG. 1 illustrates a front perspective view of an intranasal delivery device, according to an aspect of this disclosure.
FIG. 2 illustrates a back perspective view of the intranasal delivery device shown in FIG. 1.
FIG. 3 illustrates a front perspective view of an intranasal delivery device, according to another aspect of this disclosure.
FIG. 4 illustrates a back perspective view of the intranasal delivery device shown in FIG. 3.
FIG. 5 illustrates a schematic of a feedback mechanism, according to an aspect of this disclosure.
FIG. 6 illustrates a front perspective view of an intranasal delivery device, according to yet another aspect of this disclosure.
FIG. 7 illustrates a front perspective view of an intranasal delivery device, according to still another aspect of this disclosure.
FIG. 8 illustrates a back perspective view of the intranasal delivery device shown in FIG. 7.
FIG. 9 illustrates a front perspective view of an intranasal delivery device, according to another aspect of this disclosure.
FIG. 10A illustrates a back perspective view of the intranasal delivery device shown in FIG. 9 with a medicament cartridge attached.
FIG. 10B illustrates a back perspective view of the intranasal delivery device shown in FIG. 9 with a training cartridge attached.
FIG. 11 illustrates a cross-sectional side view of the intranasal delivery device shown in FIG. 9 positioned within a patient.

### DETAILED DESCRIPTION

Intranasal devices for insertion into a nasal passage for treating pain in a patient, such as pain associated with a cluster headache, are described. Unlike prior intranasal devices, the devices described herein may be used to self-administer a medicament in a home setting by a patient. The intranasal devices may be patient operated, and may be configured (programmed) based on the anatomy of the patient. The devices preferably include feedback mechanisms and controls to help the patient position the device within their nasal passage (e.g. nasal cavity).

Certain terminology is used in the description for convenience only and is not limiting. The words "proximal" and "distal" generally refer to positions or directions toward and away from, respectively, an individual or patient operating an intranasal device. The words "axial," "vertical," "transverse," "left," "right," "above," and "below" designate directions in the drawings to which reference is made. The term "substantially" is intended to mean considerable in extent or largely but not necessarily wholly that which is specified. The terminology includes the above-listed words, derivatives thereof and words of similar import.

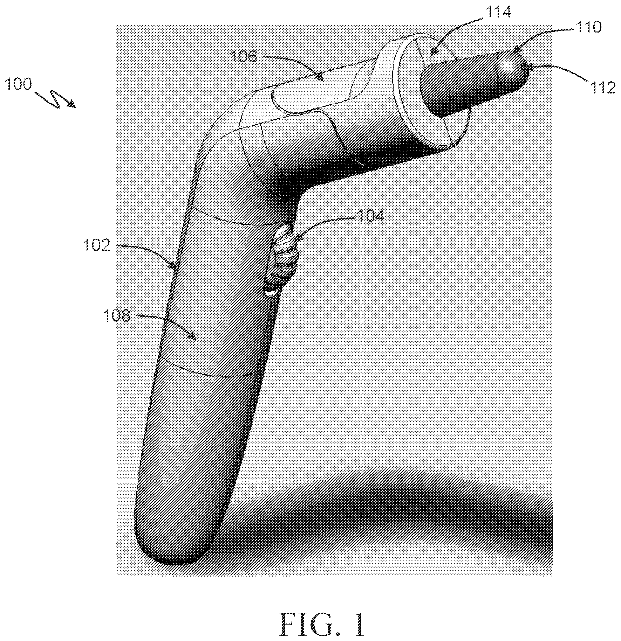

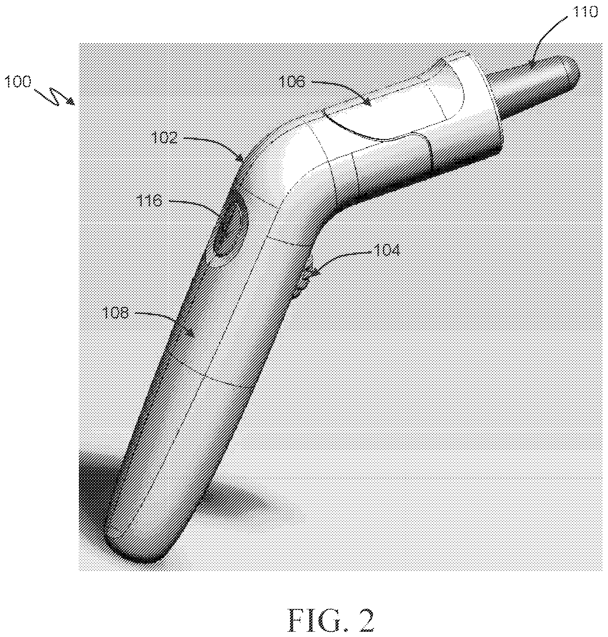

FIGS. 1 and 2 provide a front perspective view and a back perspective view, respectively, of an intranasal delivery device 100, according to a first aspect of this disclosure. The intranasal delivery device 100 includes a body 102 and a position controller 104. The body 102 has an upper portion 106 and a lower portion 108 (e.g. grip portion or handle portion). The upper portion 106 preferably extends at an angle from the lower portion 108. The upper portion 106 includes a nostril insertion tip 110 configured for entry into a nostril of the patient. While not expressly illustrated in FIGS. 1 and 2, the upper portion 106 is preferably a replaceable medicament cartridge that comprises the medicament reservoir, and preferably both the catheter and medicament reservoir. The nostril insertion tip 110 defines a channel 112 that extends from an interior of the body 102 to an exterior of the body 102. The channel 112 is configured to receive a tube (not shown) within. The tube may be, for example, a thin flexible tube such as a catheter. The catheter may be rigid, flexible, or a combination of rigid and flexible, such that the balance between rigidity and flexibility allows for advancement of the catheter into a nasal cavity without turning or getting caught. The catheter is also configured to minimize pain, discomfort, and injury during insertion. The configuration of the catheter may depend on, for example, the configuration of the delivery device to which the catheter is coupled, the method of treatment being performed, or other factors to facilitate positioning of the catheter. It will be appreciated that the embodiments of delivery devices described herein may include one or more configurations of the catheters.

The nostril insertion tip 110 may be coupled to a stopper surface 114 of the upper portion 106. The nostril insertion tip 110 may be removable, washable, replaceable, and sanitizeable. The upper surface 114 may be a substantially flat surface, and the nostril insertion tip 110 may extend substantially perpendicular from the upper surface 114. The nostril insertion tip 110 may include a single unit through which the catheter passes, or alternatively, the nostril insertion tip 110 may be telescopic and include two or more pieces which advance into the nasal cavity. The single unit insertion tip 110 may include a more rigid form so as to allow advancement of a more flexible catheter. The telescopic insertion tip 110 may include a series of one or more units which extend telescopically from the upper surface 114. The one or more units may be rigid to allow for guidance of a flexible catheter inside the nasal cavity to a desired location. In an aspect, the telescopic insertion tip 110 may be stored within the delivery device 100, such that the insertion tip 110 does not extend beyond the upper surface 114 until the device 100 is activated.

The lower portion 108 of the body 102 is configured to be gripped or held by a patient. In an aspect, the lower portion 108 is agnostic as to which hand or side the patient is using to grip the lower portion 108. The lower portion 108 includes the position controller 104, a medicament delivery control 116 (e.g. actuator), and a chamber (not visible in the figures). The position controller 104 is configured to control the movement of the catheter through the channel 112. The movement includes advancing and retracting the catheter from the body 102. In an aspect, the position controller 104 includes a thumbwheel that can be rotated by the patient to advance and retract the catheter. In an alternative aspect, the position controller 104 may also include a linear actuator, such as a motorized rack, piezoelectric motor (e.g. squiggle motor), pneumatic or hydraulic piston, or another controller configured to advance and retract a catheter. In another alternative aspect, the position controller 104 may include a small motor, such as a direct current (DC) brush or stepper motor, along with a pinch roller mechanism configured to grip the catheter to advance and retract the catheter with precision position control. The position controller 104 is preferably sized to at least partially fit within the body 102 of the device 100.

The chamber is configured to store a medicament within. The chamber may be defined by the body 102, or may be defined by a replaceable cartridge 106, the replaceable cartridge being removable from the body 102. The catheter is coupled, removably or non-removably, to the replaceable cartridge such that the catheter is in fluid communication with the chamber. In an aspect, the catheter may be attached to the replaceable cartridge such that the replaceable cartridge and the catheter are removable from the body 102 as a single unit.

The device 100 also preferably includes a feedback mechanism (not shown), a delivery system (not shown), and a power source (not shown). The feedback mechanism is configured to indicate a position of a distal end of the catheter within the nasal passage of the patient, as discussed in more detail below. The power source, such as a battery, is configured to provide power to either or all of the position controller 104, the feedback mechanism, and the delivery system.

The delivery system is operatively coupled to the chamber, and is configured to deliver the medicament from the chamber into the nasal passage via the catheter. The delivery system may include a centrifugal pump, peristaltic pump, piezoelectric pump, motorized syringe, pneumatically controlled syringe, hydraulically controlled syringe, spring or memory metal controlled syringe, a pressurized reservoir using a software controlled-valve arrangement, combinations thereof, or other delivery system configured to deliver medicament.

A method for using the intranasal delivery device 100 for administering the medicament comprises the patient aligning the nostril insertion tip 110 with one of their nostrils. The nostril insertion tip 110 may be inserted into the nostril as far it can comfortably go or up until the nostril contacts the upper surface 114 of the upper portion 106. The nostril insertion tip 110 may be made of a soft, conforming material or a material that is custom molded to the specific shape of the nostril for each patient. After inserting the tip 110, the catheter may be advanced through the channel 112 of the tip 110 and inserted into the nostril and the nasal cavity. The catheter may be advanced by a first actuation of the position controller 104, such as manually rotating the position controller 104. Alternatively, the catheter may be advanced by pressing a button or an on/off switch to activate, for example, a motor operatively coupled to the catheter. The catheter may then be positioned adjacent to or in close proximity to the sphenopalatine ganglion (SPG). The positioning of the catheter is aided by the feedback mechanism, which provides feedback to the patient indicating whether the catheter is in position or needs adjustment. For example, the feedback mechanism could indicate the position of the distal end of the catheter by using visual or audible indicators, such as light indicators, textual information, audible feedback such as tone patterns and/or synthesized speech, vibration, combinations thereof, or other indicators to inform the patient of the position of the catheter. According to an embodiment, the device is programmed with instructions from a prior training session with a trained medical professional to guide the distal end of the catheter toward the SPG.

After the catheter is positioned within the nasal cavity, the medicament is delivered from the chamber toward the SPG region. The patient may deliver the medicament through the catheter using the delivery system. The delivery system may be activated by actuating the medicament delivery control 116 (e.g. second actuation). The medicament delivery control 116 may include a button or an on/off switch that activates, for example, a motor. Alternatively, the medicament delivery control 116 may include a pump, which may be manually operated to pump the medicament into the nasal cavity. The delivery energy could also be supplied by way of a pre-pressurized drug reservoir wherein the user controls a valve to release the medicament allowing the drug to be delivered by the stored pressure. The medicament delivery control 116 may also be activated based on catheter advancement. For example, after the catheter advances a predetermined distance (e.g. minimal distance) into the nasal cavity, the delivery control 116 may be automatically activated.

After the delivery of the medicament toward the SPG, the catheter is retracted from the nasal cavity. The catheter may be retracted by the patient by manually rotating the position controller 104 in a direction opposite to the rotation for advancement. Alternatively, if the catheter is advanced by pressing a button or an on/off switch to activate, a reverse or retract button or switch may be activated by the patient to retract the catheter. Once the catheter has retracted, the nostril insertion tip 110 is removed from the nostril of the patient. The nostril insertion tip 110, the catheter, the replaceable cartridge, and any remaining medicament may be removed from the intranasal delivery device 100 and discarded.

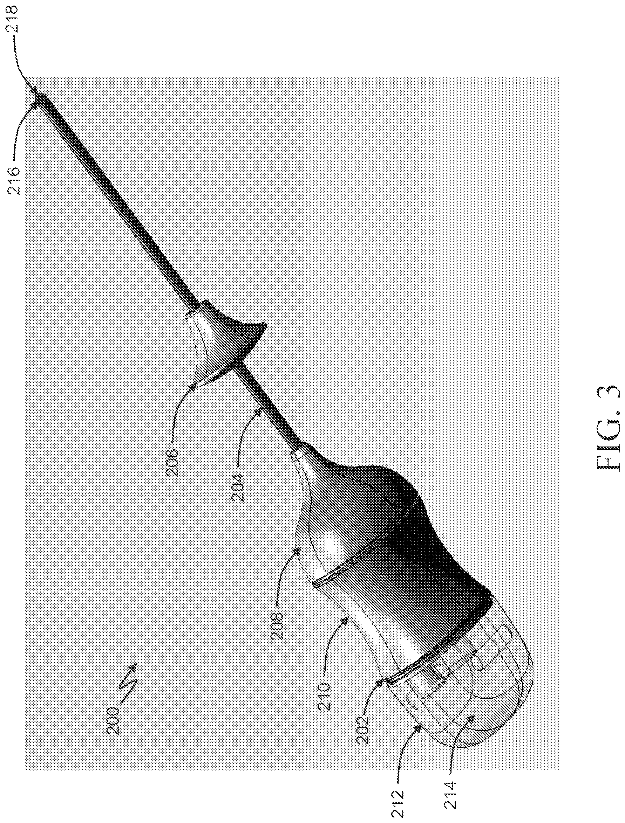

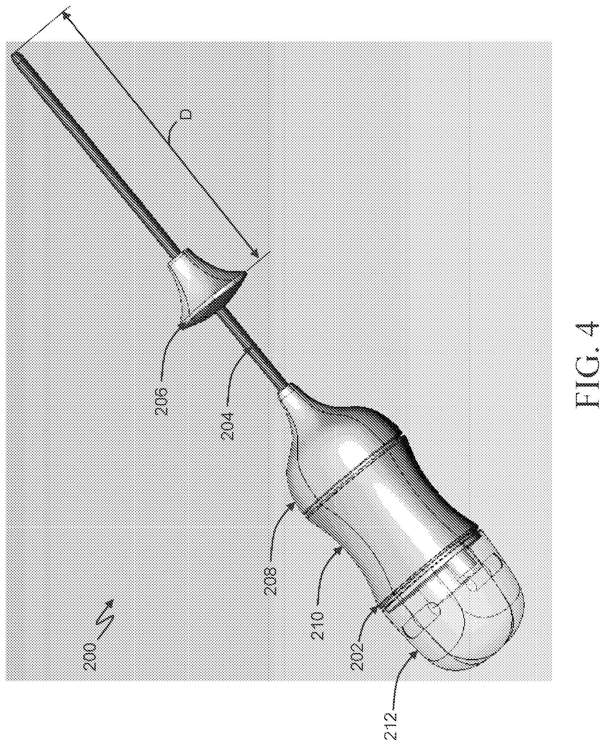

FIGS. 3 and 4 illustrate an alternate aspect of an intranasal delivery device. Portions of the aspect disclosed in FIGS. 3 and 4 are similar to aspects described above in FIGS. 1 and 2 and those portions function similarly to those described above unless specified otherwise.

FIGS. 3 and 4 provide a front perspective view and a back perspective view, respectively, of an intranasal delivery device 200, according to an aspect of this disclosure. The intranasal delivery device 200 includes a body 202, a catheter 204, and a stopper 206. The body 202 includes an upper portion 208, a middle portion 210 (e.g. grip portion or handle portion), and a lower portion 212. The upper portion 208 defines a channel (not visible in the figures) that extends from an interior of the body 202 to an exterior of the body 202. The channel is configured to receive the catheter 204 within. The middle portion 210 is positioned proximal to the upper portion 208. The middle portion 210 may be shaped or configured to be easily held by the patient. A diameter of the center of the middle portion 210 may be less than a diameter of upper and lower ends of the middle portion 210, forming a curvature that may be gripped, for example, between a thumb and index finger. In an aspect, the middle portion 210 is substantially hollow and comprises a flexible material that is compressible and expandable. The lower portion 212 of the body 202 is positioned proximal to the middle portion 210 and defines a chamber 214.

According to a first embodiment, the medicament is stored within a medicament chamber in the upper portion 208, wherein the chamber 214 is a bulb containing air that can pressurize the contents of the reservoir when the patient squeezes it. The upper portion 208 and the catheter may comprise a disposable portion that is fitted to the reusable portion (210 and 214), which may contain medicament delivery sensing and user feedback electronics. The catheter 204 is coupled, removably or non-removably, to the upper portion 208 such that the catheter 204 is in fluid communication with the medicament chamber contained within the upper portion 208.

According to a second embodiment, the chamber 214 may be configured to store the medicament within. In this embodiment, the lower portion 212 is a replaceable cartridge that may be removed from the intranasal delivery device 200 and replaced with another cartridge. The catheter 204 is coupled, removably or non-removably, to the lower portion 212 such that the catheter 204 is in fluid communication with the chamber 214. The catheter 204 extends through the upper portion 208 and the middle portion 210 of the body 202. The catheter 204 may be attached to the lower portion 212 such that the lower portion 212 and the catheter 204 are removable from the body 202 as a single unit. In an aspect, the catheter 204 may be axially fixed to the body 202, such that translation of the catheter 204 in and out of the body 202 through the channel is substantially prevented.

In other alternative embodiments, the middle portion 210 is only rigid in one plane, and flexible in the other plane. This allows pressure/squeezing in the flexible plane, and does not require the lower portion 212 to be flexible. The complete delivery device 200 may be disposable.

In accordance with the aforementioned embodiments described in connection with FIG. 3 and 4, the stopper 206 is slidably attached to the catheter 204. The position of the stopper 206 may be adjusted by translating the stopper 206 along the catheter 204 to a desired position, and may be configured based on the left or right nostril. When the desired position is achieved, the stopper 206 may be fixed or locked to the catheter 204 at that position. The desired position may be based on the anatomy of the patient, and may be determined by the prescribing physician. For example, the desired position of the stopper 206 may be based on the length and/or size of the nostril and the nasal cavity of the patient. The stopper 206 is sized to substantially prevent entry of the stopper 206 into the nasal passage.

The device 200 may also include a delivery system (not shown), a power source (not shown), and a sensor 216. The delivery system and the power source may be configured substantially similarly as the delivery system and the power source of the intranasal delivery device 100.

The stopper 206 and the sensor 216 may each compose a feedback mechanism configured to indicate a position of a distal end 218 of the catheter 204 within the nasal passage of the patient. It will be appreciated that the intranasal delivery device 200 may include either one of the stopper 206 or the sensor 216, or both the stopper 206 and the sensor 216. The stopper 206 may provide the patient with a tactile feedback. For example, the desired position of the stopper 206 on the catheter 204 may be at a position that defines a distal length (D) of the catheter 204 that is substantially the same as a distance from an opening of the nostril to the SPG region. When the patient inserts the catheter 204 into the nasal cavity, the patient will receive a tactile feedback when the stopper 206 contacts the nostril of the patient.

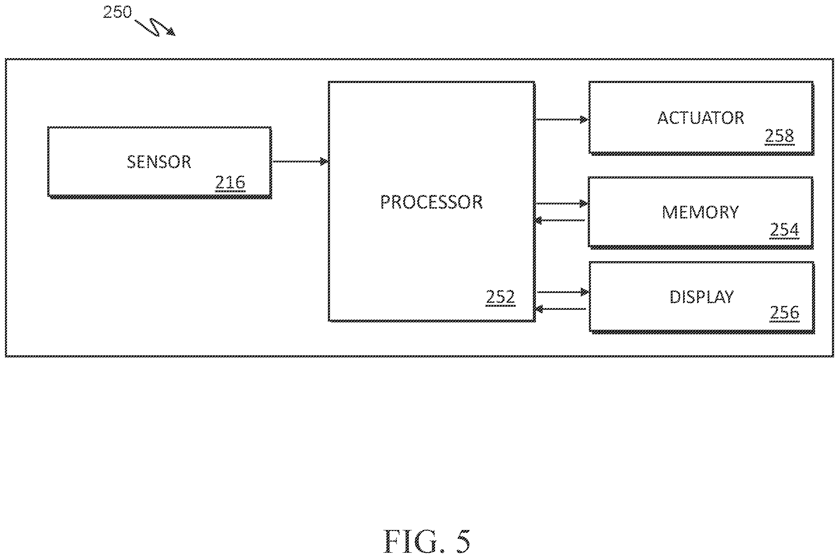

FIG. 5 illustrates a schematic of a feedback mechanism 250 that includes the sensor 216. The feedback mechanism 250 includes a controller, such as an electronic control unit, which may facilitate the positioning of the catheter 204. The feedback mechanism 250 further includes a processor 252, a memory 254, a display 256, and an actuator 258. While the feedback mechanism 250 is represented as a single unit, in other aspects the feedback mechanism 250 may be distributed as a plurality of distinct but interoperating units, incorporated into another component, or located at different locations on or off the intranasal delivery device 200.

The sensor 216 is coupled to the distal end 218 of the catheter 204. The sensor 216 may sense the position and orientation of the distal end 218 and provide feedback to the processor 252. The sensor 216 may include, for example, an Inertial Measurement Unit (IMU), which is an electronic device that integrates the functions of both a 3D accelerometer and a 3D gyroscope. The processor 252 may be configured to output signals to the actuator 258 in response to signals received from the sensor 216. The actuator 258 may include one or more actuators configured to actuate, for example, the delivery system and/or the position controller. The display 256 may also be coupled to the processor 252 to display various data to the patient relating to, for example, the position of the catheter 204 within the nasal cavity. Action may be taken in response to the data, including advancing or retracting the catheter 204. In addition to feedback during use, the processor 252 may record usage information in memory 254 and provide the usage information to a mobile device (not shown) via, for example, a wireless link over a cloud network. The usage information may also be transferred to a healthcare provider for adherence, compliance, and/or emergency use information.

The method of operating the intranasal delivery device 200 may be substantially similar to the method of operating the intranasal delivery device 100. Alternatively, a method for using the intranasal delivery device 200 for administering the medicament comprises positioning the stopper 206 on the catheter 204 at the desired position. The stopper 206 is then locked to the catheter 204 at the desired position. After the stopper 206 is locked, the patient aligns the distal end 218 of the catheter 204 with one of their nostrils, and inserts the catheter 204 into the nostril. In an aspect, the catheter 204 is axially fixed to the body 202, such that insertion of the catheter 204 involves the patient moving the entire body 202 of the intranasal delivery device 200 towards the nasal cavity so that the catheter 204 is pushed into the nasal cavity. Alternatively, the catheter 204 may be inserted using a position controller as described above. The catheter 204 is inserted into the nasal cavity until the stopper 206 contacts the nostril.

After the stopper 206 contacts the nostril, the patient may then deliver the medicament toward the SPG. Delivery of the medicament toward the SPG may be performed using a delivery system as described above. Alternatively, delivery of the medicament may be performed manually by squeezing the lower portion 212, which acts as a squeeze bulb, so that medicament is pumped out of the upper portion 208 and through the catheter 204. In this embodiment, as described above, the medicament is stored in the upper portion 208, wherein the chamber 214 is a bulb containing air that can pressurize the contents of the reservoir when the patient squeezes it.

Alternatively, the medicament may be pumped out of the chamber 214 by squeezing the middle portion 210 causing the medicament to be pushed through the catheter 204. In this aspect, the middle portion 210 functions as a squeeze bulb. In this instance, the lower portion 212 may be formed of a rigid material, such that squeezing the middle portion 210 increases the pressure within the lower portion 212 causing the medicament to be pushed out of the chamber 214. Alternatively, the lower portion 212 may be formed of a flexible material, and the medicament may be pumped through the catheter 204 by squeezing the lower portion 212. The delivery device 200 provides for consistent delivery of a medicament to a desired location regardless of variable individual application of pressure.

After the delivery of the medicament toward the SPG, the catheter 204 is retracted from the nasal cavity. The catheter may be retracted by the patient using the position controller as described above. Alternatively, the patient may manually retract the catheter 204 by pulling the body 202 of the intranasal delivery device 200 away from the nasal cavity. Once the catheter has retracted, the catheter 204 and the replaceable cartridge 212 may be removed from the body 202 and discarded. Alternatively, the entire intranasal delivery device 100 may be discarded.

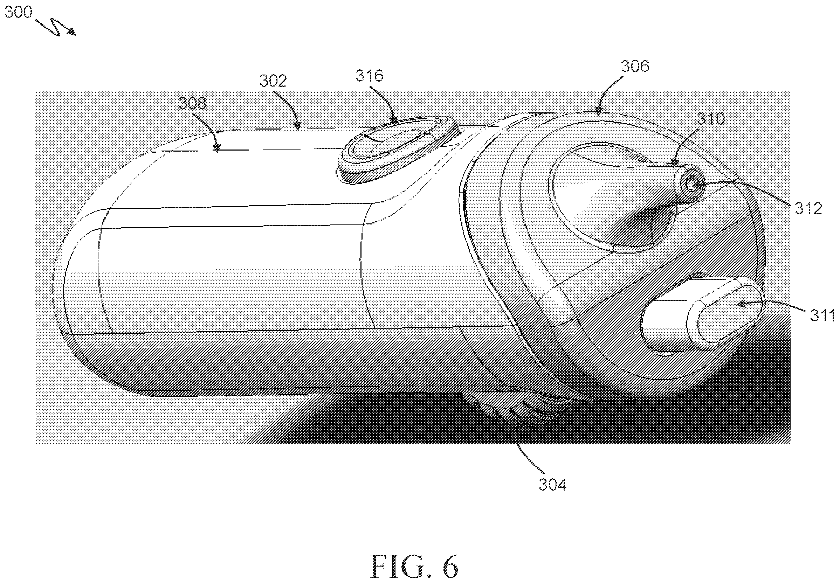

FIG. 6 illustrates another alternate aspect of an intranasal delivery device. Portions of the aspect disclosed in FIG. 6 are similar to aspects described above in FIGS. 1 through 5 and those portions function similarly to those described above unless specified otherwise.

FIG. 6 provides a front perspective view of an intranasal delivery device 300, according to an aspect of this disclosure. The intranasal delivery device 300 includes a body 302 and a position controller 304. The body 302 has an upper portion 306 and a lower portion 308 (e.g. grip portion or handle portion). The upper portion 306 includes a nostril insertion tip 310 and an interlock switch 311. Preferably, the upper portion 306 is a replaceable cartridge that also contains the medicament chamber and catheter. The nostril insertion tip 310 is configured for entry into a nostril of the patient. The nostril insertion tip 310 defines a channel 312 that extends from an interior of the body 302 to an exterior of the body 302. The channel 312 is configured to receive a catheter (not shown) within. In an aspect, the nostril insertion tip 310 of the intranasal delivery device 300 may be configured substantially similarly to the nostril insertion tip 110 of the intranasal delivery device 100.

The lower portion 308 of the body 302 is configured to be gripped or held by the patient. The lower portion 308 includes the position controller 304 and a medicament delivery control 316 (not visible in the figures). The medicament chamber of the upper portion 306 (not visible in the figures) is preferably disposed within the lower portion 308. The position controller 304, the medicament delivery control 316, and the chamber of the intranasal delivery device 300 may be configured substantially similarly to the position controller 104, the medicament delivery control 116, and the chamber of the intranasal delivery device 100, respectively.

The device 400 may also include a feedback mechanism (not shown) and a power source (not shown) configured substantially similarly to the feedback mechanisms and power sources of intranasal delivery devices 100, 200, 300, and 400 described above.

The method of operating the intranasal delivery device 300 may be substantially similar to the method of operating either of the intranasal delivery devices 100 and 200 described above. Additionally, during the insertion of the nostril insertion tip 310 into the nostril of the patient, the interlock switch 311 may provide an indication to the patient that the intranasal delivery device 300 has been inserted fully into the nostril. In an aspect, the interlock switch 311 may be locked, and released only when the catheter is in the desired location. The interlock switch 311 may be spring loaded, and configured to compress against the upper lip of the patient.

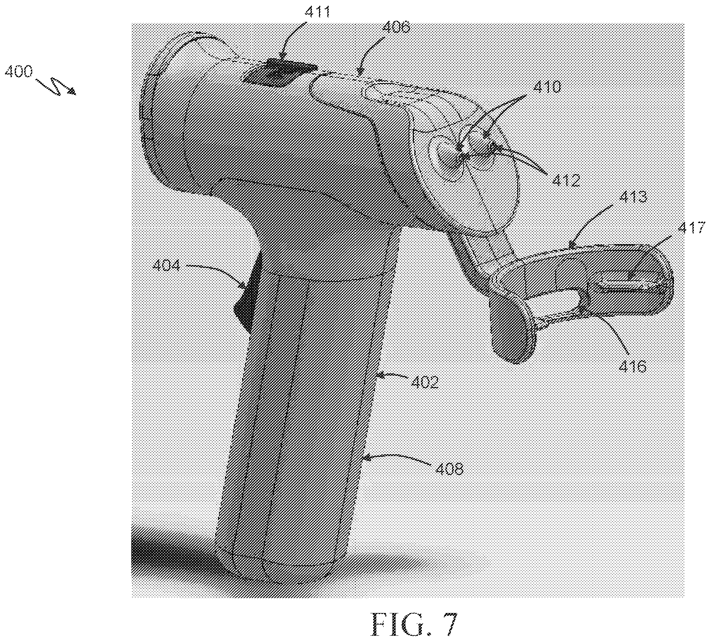

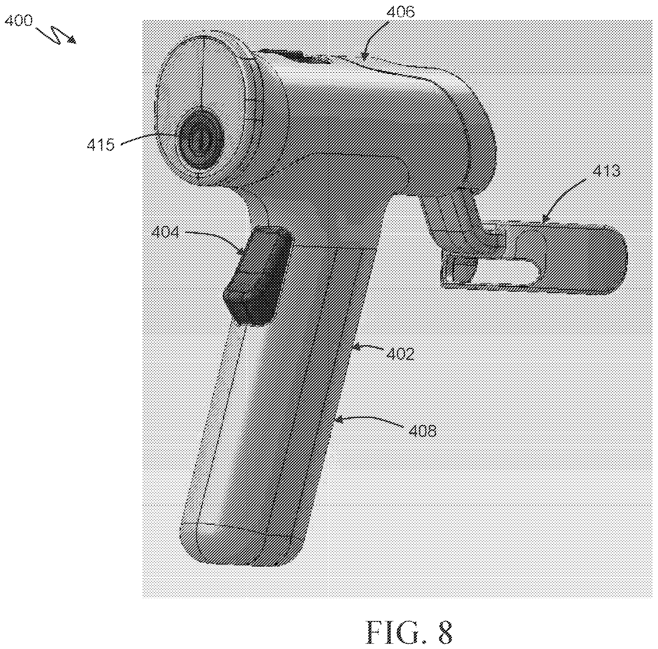

FIGS. 7 and 8 illustrate another alternate aspect of an intranasal delivery device. Portions of the aspect disclosed in FIGS. 7 and 8 are similar to aspects described above in FIGS. 1 through 6 and those portions function similarly to those described above unless specified otherwise.

FIGS. 7 and 8 provide a front perspective view and a back perspective view, respectively, of an intranasal delivery device 400, according to an aspect of this disclosure. The intranasal delivery device 400 includes a body 402 and a controller 404. The body 402 has an upper portion 406 and a lower portion 408 (e.g. grip portion or handle portion). The upper portion 406 is preferably a replaceable cartridge comprising the medicament chamber and catheter(s). The upper portion 406 includes a pair of insertion tips 410, an advance/retract switch 404, a mouth member 413 (e.g. bite block), and a power control 415. The mouth member 413 is configured to contact a mouth of the patient operating the device 400, and may include a breathing window 416, a teeth shelf 417, or other features to facilitate positioning of the delivery device 400. The mouth member 413 may be adjustable based on the anatomy of the patient. The mouth member 413 may be custom molded to the patient and preferably remains as part of the reusable body. The mouth member 413 may be configured to be removed and sanitized. The power control 415 is configured to actuate a power source of the device 400 between an "on" position and an "off' position.

The insertion tips 410 are configured to align with each nostril of the patient. The insertion tips 410 define channels 412 that extend from an interior of the body 402 to an exterior of the body 402. The channels 412 are each configured to receive a catheter (not shown) within. In an aspect, each of the insertion tips 410 of the intranasal delivery device 400 may be configured substantially similarly to the nostril insertion tips 110 and 310 of the intranasal delivery devices 100 and 300.

The lower portion 408 of the body 402 is configured to be gripped or held by the patient. The lower portion 408 includes the controller 404. The medicament chamber contained in the upper portion 406 is preferably disposed within the lower portion 408. The chamber is configured to store a medicament within, as described above. One or two catheters may be coupled to the chamber, such that each catheter is in fluid communication with a medicament stored within the chamber. Each of the one or two catheters extend from the chamber and through a respective channel 412.

The controller 404 is configured to control a position controller to move the catheters through the channels 412, and further configured to control a delivery system to deliver the medicament from the chamber into the nasal cavity via the one or two catheters. The position controller and the delivery system may be configured substantially similarly to the position controllers and delivery systems of the intranasal delivery devices 100, 200, and 300. The controller 404 may comprise a "trigger," that may be actuated by the patient while holding the lower portion 408 of the body 402. Switch 411 is used to select which side (left or right) is treated. Advancement and retraction of a single catheter along with dispensing of the medicament are preferably automatic in this embodiment once the patient presses and holds the trigger switch. Treatment is preferably applied one side at a time.

The device 400 may also include a feedback mechanism (not shown) and a power source (not shown) configured substantially similarly to the feedback mechanisms and power sources of intranasal delivery devices 100 and 200 described above.

The method of operating the intranasal delivery device 400 may be substantially similar to the methods of operating any of the intranasal delivery devices 100, 200, and 300 described above. Additionally, during alignment of the insertion tips 410 with each respective nostril, the patient also aligns the mouth member 413 with their mouth. In alternative aspects, the mouth member 413 may be aligned with, for example, the patient's teeth and/or chin. The patient may grip the mouth member 413 with their teeth. After alignment of the insertion tips 410 and the mouth member 413, the intranasal delivery device 400 may be operated as discussed above.

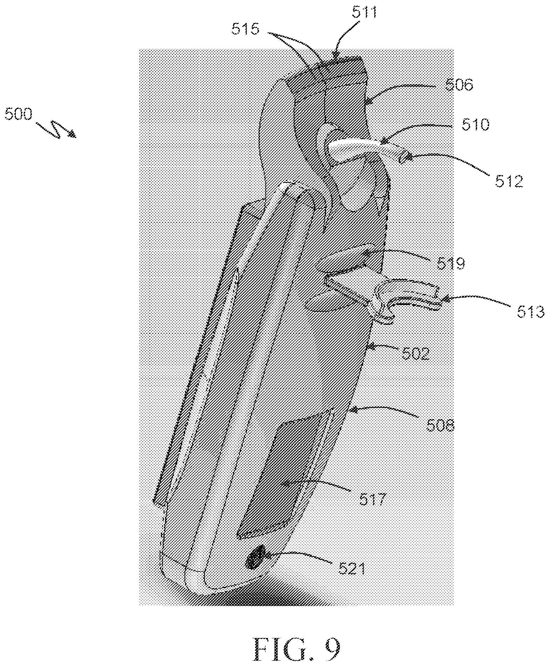

FIGS. 9-10B illustrate another alternate aspect of an intranasal delivery device. Portions of the aspect disclosed in FIGS. 9-10B are similar to aspects described above in FIGS. 1 through 8 and those portions function similarly to those described above unless specified otherwise.

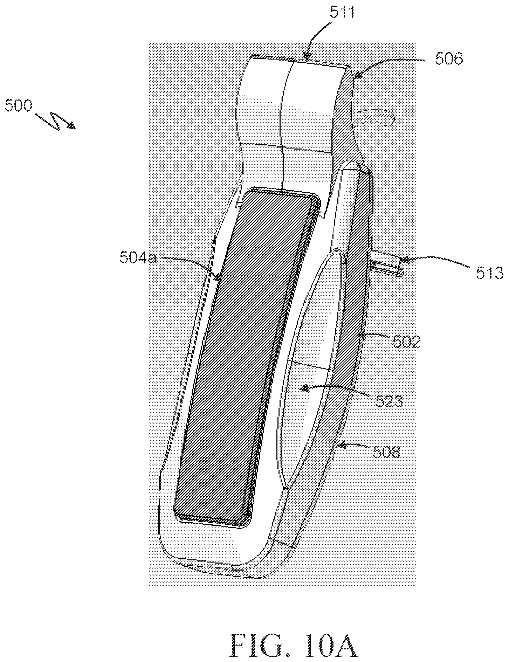

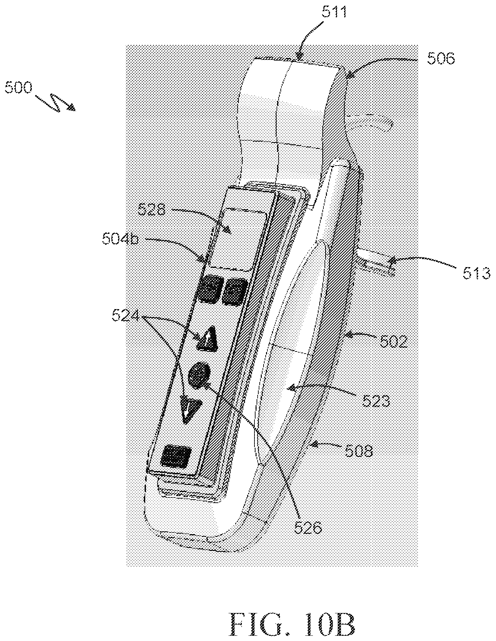

FIG. 9 provides a front perspective view, FIG. 10A provides a back perspective view with a medicament cartridge attached, and FIG. 10B provides a back perspective view with a training cartridge attached, respectively, of an intranasal delivery device 500, according to an aspect of this disclosure. The intranasal delivery device 500 includes a body 502 and a replaceable medicament cartridge 504a or training cartridge 504b. The body 502 has an upper portion 506 and a lower portion 508 (e.g. grip portion or handle portion). The upper portion 506 may extend at an angle from the lower portion 508 and/or be rotatable relative to the lower portion 508. The upper portion 506 includes a nostril insertion tip 510 and a nose stop 511. The nostril insertion tip 510 is configured for entry into a nostril of the patient, and may be rotatable (e.g. ball joint) to align with a respective nostril. The nostril insertion tip 510 defines a channel 512 that extends from an interior of the body 502 to an exterior of the body 502. The channel 512 is configured to receive a catheter 501 within. In an aspect, the nostril insertion tip 510 of the intranasal delivery device 500 may be configured substantially similarly to the nostril insertion tips 110 and 310 of the intranasal delivery devices 100 and 300.

The nose stop 511 includes at least one light emitting diode (LED) 515. The nose stop 511 is configured to provide feedback to the patient during use of the intranasal delivery device 500 regarding the position and/or path of the catheter within the nasal cavity. For example, the at least one LED 515 may include different colored lights. One light may light when inserting the catheter, and once the catheter reaches a desired position, the light may turn off and another light may turn on, indicating to the patient that the catheter is positioned properly.

The lower portion 508 of the body 502 is configured to be gripped or held by the patient. The lower portion 508 includes the replacement medicament cartridge 504a (FIG. 10A) or the training cartridge 504b (FIG. 10b), a mouth member 513 (e.g. bite block), an liquid crystal display (LCD) display 517, a lip recess 519, a power control 521, and a grip recess 523. The mouth member 513 and the power control 521 may be configured substantially similarly to the mouth member 413 and the power control 415 of the intranasal delivery device 400, respectively. The LCD display 517 may be configured substantially similarly to the display 256 of the intranasal delivery device 200. The lip recess 519 is configured to contact a lip of the patient when the mouth member 513 is positioned proximate to the mouth of the patient. The grip recess 525 is configured for ambidextrous gripping of the lower portion 508

The replaceable medicament cartridge 504a and the training cartridge 504b are removable from the intranasal delivery device 500. The replaceable medicament cartridge 504a includes a chamber (not visible in the figures), and the training cartridge 504b may include a chamber, a position controller 524 and a medicament delivery control 526. The position controller 524, the medicament delivery control 526, and the chamber of the intranasal delivery device 500 may be configured substantially similarly to the position controllers 104, 304, and 404, the medicament delivery control 116, 316, and the chambers of the intranasal delivery device 100, 300, and 400, respectively. FIG. 10B shows an embodiment of a device that may be used during a training session by a physician, which includes a display 528.

The device 500 may also include a feedback mechanism (not shown), a delivery system (not shown), and a power source (not shown) configured substantially similarly to the feedback mechanisms, delivery systems, and power sources of intranasal delivery devices 100, 200, 300, and 400 described above.

It will be appreciated that the embodiments of delivery devices 100, 200, 300, 400, and 500 described herein may include one or more configurations of catheters. For example, each delivery device 100, 200, 300, 400, and 500 may include a catheter that is either rigid, flexible, or a combination of rigid and flexible, such that the balance between rigidity and flexibility allows for advancement of the catheter into a nasal cavity without turning or getting caught, as described in detail above.

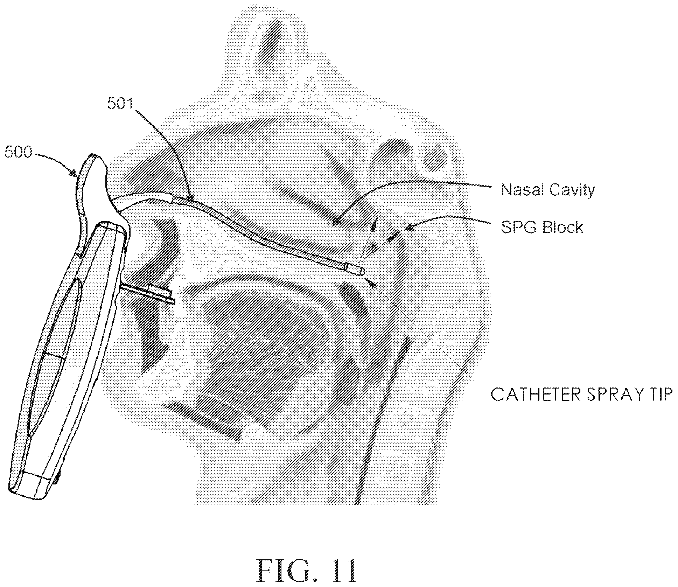

The method of operating the intranasal delivery device 500 may be substantially similar to the methods of operating any of the intranasal delivery devices 100, 200, 300, and 400 described above. FIG. 11 illustrates the intranasal delivery device 500 in an inserted position with the catheter 501 extended within the nasal cavity.

The intranasal delivery devices described herein are ergonomically designed so that the patient can comfortably hold and maintain a position of the device at the nostril during self-administration of a medicament. The patient does not have to know how far or how hard to insert and push the catheter to reach the desired position, which prevents injury. An advantage of a motorized intranasal delivery device is safety. The possibility of injury is greatly reduced with speed, position, and force feedback provided by a feedback mechanism in real time. For example, if excessive insertion force is detected, the device may be configured to stop advancement of the catheter and/or retract the catheter. The device may then provide information to the patient regarding a corrective action. Speed of inserting the catheter may also be controlled to avoid tissue injury.

An additional embodiment of the present invention is a kit comprising: (a) an intranasal device as described herein (optionally without the medicament chamber attached to the device) and (b) one or more replaceable cartridges, each replaceable cartridge comprising the chamber comprising the medicament. According to another embodiment, a kit comprises (a) an intranasal device as described herein (optionally without the medicament chamber and catheter attached to the device) and (b) one or more replaceable cartridges, each replaceable cartridge comprising the catheter and the chamber comprising the medicament. According to another embodiment, a kit comprises (a) an intranasal device as described herein (optionally without the medicament chamber and catheter attached to the device), (b) one or more replaceable cartridges, each replaceable cartridge comprising the chamber comprising the medicament, and (c) one or more catheters. The patient then assembles components of the kit before self-administering medicament; for example, by attaching the replaceable cartridge to the intranasal device. Each kit may include one or more replaceable cartridges comprising the medicament chamber and optionally one or more catheters (if the catheter is not included as part of the replaceable cartridge) to be used with the device for multiple dosing sessions. Each kit may also include one or more sanitizing wipes in a single package or individually packed.

The scope of protection of the present invention is defined by the appended claims.

## Claims

1. An intranasal device (200) for self-administration of a therapeutically effective amount of medicament toward the SPG, the intranasal device comprising:
a body (202) having a chamber configured to store the medicament;
a catheter (204) configured to extend from the body, a distal end of the catheter being configured for entry into a nasal passage of a patient, the catheter being in fluid communication with the chamber;
a feedback mechanism (206) configured to indicate a position of the distal end of the catheter within the nasal passage; and
a delivery system configured to deliver the medicament from the chamber into the nasal passage via the catheter,
wherein the delivery system comprises a centrifugal pump, a peristaltic pump, a piezoelectric pump, a motorized syringe, a pneumatically controlled syringe, a hydraulically controlled syringe, a pressurized reservoir using a software controlled-valve arrangement or combinations thereof.

2. The intranasal device of claim 1, wherein the chamber is defined by a replaceable cartridge, the replaceable cartridge being removable from the body, preferably wherein the catheter is coupled to the replaceable cartridge such that the replaceable cartridge and the catheter are removable from the body as a single unit.

3. The intranasal device of claim 1, wherein the catheter is axially fixed within a channel of the body.

4. The intranasal device of claim 1, further comprising a position controller coupled to the body, the position controller being configured to advance and retract the catheter from the body.

5. The intranasal device of claim 1, wherein the delivery system comprises a pump configured to deliver the medicament from the chamber into the nasal passage via the catheter.

6. The intranasal device of claim 5, further comprising an actuator configured to be manipulated by the patient, the actuator causing the delivery of the medicament from the chamber into the nasal passage via the catheter.

7. The intranasal device of claim 1, further comprising a handle configured to be gripped by the patient when the distal end of the catheter enters into the nasal passage of the patient and the medicament is delivered from the chamber into the nasal passage via the catheter.

8. The intranasal device of claim 1, wherein the feedback mechanism is further configured to visually or audibly indicate the position of the distal end of the catheter within the nasal passage.

9. The intranasal device of claim 1, wherein the intranasal device is used to treat a cluster headache using a therapeutically effective amount of the medicament.

10. The intranasal device of claim 1, wherein the medicament comprises lidocaine or a pharmaceutically acceptable salt thereof.

11. A kit comprising: (a) the intranasal device of any of claims 1 to 9 and (b) one or more replaceable cartridges, each replaceable cartridge comprising the chamber comprising the medicament.

12. A kit comprising: (a) the intranasal device of any of claims 1 to 9 and (b) one or more replaceable cartridges, each replaceable cartridge comprising the catheter and the chamber comprising the medicament.

13. The intranasal device of any of claims 1 to 9, wherein the device is programmed according to the patient's anatomy to guide the distal end of the catheter toward the SPG.

14. The kit of any of claims 11 or 12, wherein the device is programmed according to the patient's anatomy to guide the distal end of the catheter toward the SPG.

15. The intranasal device of any of claims 1 to 9, wherein the medicament comprises one or more pharmaceutically active compounds selected from the group comprising or consisting of : lidocaine, zolmitriptan, sumatriptan, ambucaine, amolanone, amylocaine, benoxinate, betoxycaine, biphenamine, bupivacaine, butacaine, butamben, butanilicicaine, butethamine, butoxycaine, carticaine, cocaethylene, cocaine, cyclomethycaine, dibucaine, dimethisoquin, dimethocaine, diperodon, dyclonine, ecgonidine, ecgonine, ethyl aminobenzoate, ethyl chloride, etidocaine, eucaine, euprocin, fenalcomine, fomocaine, hexylcaine, hydroxyprocaine, hydroxytetracaine, isobutyl p-aminobenzoate, leucinocaine mesylate, levoxadrol, meperidine, mepivacaine, meprylcaine, metabutoxycaine, methyl chloride, myrtecaine, naepaine, octacaine, orthocaine, oxethazaine, parethoxycaine, phenacaine, phenol, a pipecoloxylidide, piperocaine, piridocaine, polidocanol, pramoxine, sameridine, prilocaine, propanocaine, proparacaine, propipocaine, propoxycaine, pseudococaine, pyrrocaine, quinine urea, risocaine, ropivacaine, salicyl alcohol, tetracaine, tolycaine, trimecaine, veratridine, zolamine, pharmaceutically acceptable salts thereof, and combinations thereof, for use in the self-treatment of pain.

## Patentansprüche

1. Intranasale Vorrichtung (200) zur Selbstverabreichung einer therapeutisch wirksamen Menge eines Medikaments an den SPG, wobei die intranasale Vorrichtung umfasst:
einen Körper (202) mit einer Kammer, gestaltet zum Enthalten des Medikaments;
einen Katheter (204), gestaltet zum Ausgehen von dem Körper, wobei ein distales Ende des Katheters zum Eintreten in einen Nasengang eines Patienten gestaltet ist, wobei der Katheter in Fluidverbindung mit der Kammer steht;
einen Rückmeldungsmechanismus (206), gestaltet zum Anzeigen einer Position des distalen Endes des Katheters in dem Nasengang; und
ein Abgabesystem, gestaltet zum Abgeben des Medikaments aus der Kammer über den Katheter in den Nasengang,
wobei das Abgabesystem eine Zentrifugalpumpe, eine peristaltische Pumpe, eine piezoelektrische Pumpe, eine motorisierte Spritze, eine pneumatisch gesteuerte Spritze, eine hydraulisch gesteuerte Spritze, ein druckbeaufschlagtes Reservoir mit einer softwaregesteuerten Ventilanordnung oder Kombinationen davon umfasst.

2. Intranasale Vorrichtung gemäß Anspruch 1, wobei die Kammer durch eine ersetzbare Kartusche definiert ist, wobei die ersetzbare Kartusche von dem Körper entfernbar ist, wobei der Katheter vorzugsweise so an die ersetzbare Kartusche gekoppelt ist, dass die ersetzbare Kartusche und der Katheter als eine einzige Einheit von dem Körper entfernbar sind.

3. Intranasale Vorrichtung gemäß Anspruch 1, wobei der Katheter innerhalb eines Kanals des Körpers axial fixiert ist.

4. Intranasale Vorrichtung gemäß Anspruch 1, ferner umfassend eine Positionssteuerung, die an den Körper gekoppelt ist, wobei die Positionssteuerung dafür gestaltet ist, den Katheter in den Körper vorzuschieben und daraus zurückzuziehen.

5. Intranasale Vorrichtung gemäß Anspruch 1, wobei das Abgabesystem eine Pumpe umfasst, die dafür gestaltet ist, das Medikament aus der Kammer über den Katheter in den Nasengang abzugeben.

6. Intranasale Vorrichtung gemäß Anspruch 5, ferner umfassend einen Aktor, der dafür gestaltet ist, von dem Patienten manipuliert zu werden, wobei der Aktor die Abgabe des Medikaments aus der Kammer über den Katheter in den Nasengang bewirkt.

7. Intranasale Vorrichtung gemäß Anspruch 1, ferner umfassend einen Griff, der dafür gestaltet ist, von dem Patienten gegriffen zu werden, wenn das distale Ende des Katheters in den Nasengang des Patienten eintritt und das Medikament aus der Kammer über den Katheter in den Nasengang abgegeben wird.

8. Intranasale Vorrichtung gemäß Anspruch 1, wobei der Rückmeldungsmechanismus ferner dafür gestaltet ist, die Position des distalen Endes des Katheters in dem Nasengang sichtbar oder hörbar anzuzeigen.

9. Intranasale Vorrichtung gemäß Anspruch 1, wobei die intranasale Vorrichtung zur Behandlung eines Cluster-Kopfschmerzes unter Verwendung einer therapeutisch wirksamen Menge des Medikaments verwendet wird.

10. Intranasale Vorrichtung gemäß Anspruch 1, wobei das Medikament Lidocain oder ein pharmazeutisch annehmbares Salz davon umfasst.

11. Kit umfassend: (a) die intranasale Vorrichtung gemäß einem der Ansprüche 1 bis 9 und (b) eine oder mehrere ersetzbare Kartuschen, wobei jede ersetzbare Kartusche die Kammer umfassend das Medikament umfasst.

12. Kit umfassend: (a) die intranasale Vorrichtung gemäß einem der Ansprüche 1 bis 9 und (b) eine oder mehrere ersetzbare Kartuschen, wobei jede ersetzbare Kartusche den Katheter und die Kammer umfassend das Medikament umfasst.

13. Intranasale Vorrichtung gemäß einem der Ansprüche 1 bis 9, wobei die Vorrichtung der Anatomie des Patienten entsprechend programmiert ist, um das distale Ende des Katheters zu dem SPG zu führen.

14. Kit gemäß einem der Ansprüche 11 oder 12, wobei die Vorrichtung der Anatomie des Patienten entsprechend programmiert ist, um das distale Ende des Katheters zu dem SPG zu führen.

15. Intranasale Vorrichtung gemäß einem der Ansprüche 1 bis 9, wobei das Medikament einen oder mehrere pharmazeutische Wirkstoffe umfasst, ausgewählt aus der Gruppe umfassend oder bestehend aus: Lidocain, Zolmitriptan, Sumatriptan, Ambucain, Amolanon, Amylocain, Benoxinat, Betoxycain, Biphenamin, Bupivacain, Butacain, Butamben, Butanilicicain, Butethamin, Butoxycain, Carticain, Cocaethylen, Cocain, Cyclomethycain, Dibucain, Dimethisoquin, Dimethocain, Diperodon, Dyclonin, Ecgonidin, Ecgonin, Ethylaminobenzoat, Ethylchlorid, Etidocain, Eucain, Euprocin, Fenalcomin, Fomocain, Hexylcain, Hydroxyprocain, Hydroxytetracain, Isobutyl-p-aminobenzoat, Leucinocainmesylat, Levoxadrol, Meperidin, Mepivacain, Meprylcain, Metabutoxycain, Methylchlorid, Myrtecain, Naepain, Octacain, Orthocain, Oxethazain, Parethoxycain, Phenacain, Phenol, einem Pipecoloxylidid, Piperocain, Piridocain, Polidocanol, Pramoxin, Sameridin, Prilocain, Propanocain, Proparacain, Propipocain, Propoxycain, Pseudococain, Pyrrocain, Chinin-Harnstoff, Risocain, Ropivacain, Salicylalkohol, Tetracain, Tolycain, Trimecain, Veratridin, Zolamin, pharmazeutisch annehmbaren Salzen davon und Kombinationen davon, zur Verwendung bei der Selbstbehandlung von Schmerz.

## Revendications

1. Dispositif intranasal (200) pour l'autoadministration d'une quantité thérapeutiquement efficace de médicament vers le ganglion sphénopalatin (SPG), le dispositif intranasal comprenant :
un corps (202) comportant une chambre configurée pour stocker le médicament ;
un cathéter (204) configuré pour s'étendre à partir du corps, une extrémité distale du cathéter étant configurée pour entrer dans un passage nasal d'un patient, le cathéter étant en communication fluidique avec la chambre ;
un mécanisme de rétroaction (206) configuré pour indiquer une position de l'extrémité distale du cathéter dans le passage nasal ; et
un système d'administration configuré pour administrer le médicament depuis la chambre dans le passage nasal par le biais du cathéter,
dans lequel le système d'administration comprend une pompe centrifuge, une pompe péristaltique, une pompe piézoélectrique, une seringue motorisée, une seringue à commande pneumatique, une seringue à commande hydraulique, un réservoir sous pression utilisant un agencement de valves commandé par logiciel ou des combinaisons de ceux-ci.

2. Dispositif intranasal selon la revendication 1, dans lequel la chambre est définie par une cartouche remplaçable, la cartouche remplaçable étant amovible depuis le corps, de préférence dans lequel le cathéter est accouplé à la cartouche remplaçable de telle sorte que la cartouche remplaçable et le cathéter soient amovibles depuis le corps en tant qu'unité unique.

3. Dispositif intranasal selon la revendication 1, dans lequel le cathéter est fixé axialement dans un canal du corps.

4. Dispositif intranasal selon la revendication 1, comprenant en outre un dispositif de commande de position accouplé au corps, le dispositif de commande de position étant configuré pour avancer et rétracter le cathéter depuis le corps.

5. Dispositif intranasal selon la revendication 1, dans lequel le système d'administration comprend une pompe configurée pour administrer le médicament depuis la chambre dans le passage nasal par le biais du cathéter.

6. Dispositif intranasal selon la revendication 5, comprenant en outre un actionneur configuré pour être manipulé par le patient, l'actionneur provoquant l'administration du médicament depuis la chambre dans le passage nasal par le biais du cathéter.

7. Dispositif intranasal selon la revendication 1, comprenant en outre une poignée configurée pour être saisie par le patient lorsque l'extrémité distale du cathéter entre dans le passage nasal du patient et que le médicament est administré depuis la chambre dans le passage nasal par le biais du cathéter.

8. Dispositif intranasal selon la revendication 1, dans lequel le mécanisme de rétroaction est en outre configuré pour indiquer de façon visuelle ou sonore la position de l'extrémité distale du cathéter dans le passage nasal.

9. Dispositif intranasal selon la revendication 1, dans lequel le dispositif intranasal est utilisé pour traiter une algie vasculaire de la face à l'aide d'une quantité thérapeutiquement efficace du médicament.

10. Dispositif intranasal selon la revendication 1, dans lequel le médicament comprend de la lidocaïne ou un sel pharmaceutiquement acceptable de celle-ci.

11. Kit comprenant : (a) le dispositif intranasal selon l'une quelconque des revendications 1 à 9 et (b) une ou plusieurs cartouches remplaçables, chaque cartouche remplaçable comprenant la chambre contenant le médicament.

12. Kit comprenant : (a) le dispositif intranasal selon l'une quelconque des revendications 1 à 9 et (b) une ou plusieurs cartouches remplaçables, chaque cartouche remplaçable comprenant le cathéter et la chambre comprenant le médicament.

13. Dispositif intranasal selon l'une quelconque des revendications 1 à 9, dans lequel le dispositif est programmé en fonction de l'anatomie du patient pour guider l'extrémité distale du cathéter vers le SPG.

14. Dispositif intranasal selon l'une quelconque des revendications 11 ou 12, dans lequel le dispositif est programmé en fonction de l'anatomie du patient pour guider l'extrémité distale du cathéter vers le SPG.

15. Dispositif intranasal selon l'une quelconque des revendications 1 à 9, dans lequel le médicament comprend un ou plusieurs composés pharmaceutiquement actifs choisis dans le groupe comprenant ou constitué par : lidocaïne, zolmitriptan, sumatriptan, ambucaïne, amolanone, amylocaïne, bénoxinate, bétoxycaïne, biphénamine, bupivacaïne, butacaïne, butamben, butanilicicaine, butéthamine, butoxycaïne, carticaïne, cocaéthylène, cocaïne, cyclométhycaïne, dibucaïne, diméthisoquine, diméthocaïne, diperodon, dyclonine, ecgonidine, ecgonine, aminobenzoate d'éthyle, chlorure d'éthyle, étidocaïne, eucaïne, euprocine, fenalcomine, fomocaïne, hexylcaïne, hydroxyprocaine, hydroxytétracaïne, p-aminobenzoate d'isobutyle, mésylate de leucinocaïne, levoxadrol, mépéridine, mépivacaïne, méprylcaïne, métabutoxycaïne, chlorure de méthyle, myrtecaïne, naepaïne, octacaïne, orthocaïne, oxéthazaïne, paréthoxycaïne, phénacaïne, phénol, pipécoloxylidide, pipérocaïne, piridocaïne, polidocanol, pramoxine, saméridine, prilocaïne, propanocaïne, proparacaïne, propipocaïne, propoxycaïne, pseudococaïne, pyrrocaïne, quinine urée, risocaïne, ropivacaine, alcool salicylique, tétracaïne, tolycaïne, trimécaïne, vératridine, zolamine, des sels pharmaceutiquement acceptables de ceux-ci et des combinaisons de ceux-ci, pour une utilisation dans l'autotraitement de la douleur.
